# EUROPEAN PATENT SPECIFICATION

(11) **EP 1 434 076 B1**
(45) Date of publication and mention of the grant of the patent: **24.01.2007**
(21) Application number: 03258201.7
(22) Date of filing: 23.12.2003
(51) Int. Cl.: G02B 21/24, F16M 11/06, F16M 11/08, F16M 11/12, F16M 11/02, A61B 6/00

(54) **A rotary support structure for medical equipment**
Rotierende Trägerstruktur für medizinische Ausrüstung
Dispositif de support rotatif pour équipement médical

(30) Priority: 27.12.2002 JP 2002380411
(43) Date of publication of application: 30.06.2004
(73) Proprietor: MITAKA KOHKI CO., LTD., Mitaka-shi, Tokyo (JP)
(72) Inventor: Nakamura, Katsushige Mitaka Kohki Co., Ltd, Mitaka-shi Tokyo (JP)
(74) Representative: Skone James, Robert Edmund

(56) References cited:
- US-A- 5 784 435
- US-A- 5 812 301
- US-B1- 6 338 566

## Description

The present invention relates to a rotary support structure for medical equipment such as a surgical microscope with a stand system.

In the field of brain surgery, heart surgery, etc., precise operations are performed observing diseased parts under magnification by a microscope. Since a surgical microscope is a heavy piece of equipment, it is supported in a pendent state at an end of a support arm of a stand system, and can be moved to any aerial location and stopped at the selected location by means of a balancing mechanism with a counterweight.

A vertical location of an end member being provided at the end of the support arm can be maintained by means of a linking mechanism even when moving the support arm. Under the end member a rotary body is provided, the rotary body being rotatable in a lateral direction about a vertical axis of rotation passing through the end member, and the surgical microscope is supported with the rotary body.

Consequently, a direction of observation by the surgical microscope can be freely changed by means of rotating the surgical microscope horizontally together with the rotary body about the vertical axis of rotation.

For an operating surgeon, it is preferable that an angle of rotation around the axis of rotation of the surgical microscope is as large as possible and most preferable that the microscope is rotatable in all directions without limit. Thereby, a direction of the surgical microscope can be sequentially changed during the process of an operation.

However, since wirings for supplying electricity or signals are inserted through a rotary support portion of the surgical microscope, the wirings are liable to become tangled in one another or twisted together resulting from accumulation of angles of rotation due to unlimited rotation of the surgical microscope, because a history of rotations of 2nπ radians (*n* is an integer) cannot be distinguished externally.

Therefore, a stopping mechanism has conventionally been disposed between a support arm and a rotary body in order for the rotary body not to rotate more than 1 turn (360°). Although the inner wirings can be twisted a few turns without any problem, the stopping mechanism by which rotation is restricted to less than 1 turn has been employed in order to protect the wirings.

Since an angle of rotation of a surgical microscope has been less than one turn, protection of inner wirings has been achieved but a range of rotational angle has been insufficient for surgeons. Therefore, there has been need for a proposal for a stopping mechanism by which rotation is made possible for more than 1 turn but is stopped after a few turns for the protection of the wirings.

US-A-5784435 describes a support column for an X-ray tube in which rotation through an angle greater than 360 degrees is permitted by utilising a rotational reference point in conjunction with a rotation plate.

According to a first technical aspect of the present invention, it is characterized that a rotary support structure for medical equipment, in which a rotary body rotatable about a theoretical axis of rotation is attached onto an end member of a support arm of a stand system and on which the medical equipment is supported by the rotary body, comprising: rotation control means being disposed between the end member and the rotary body and being rotatable about the axis of rotation; a first linking groove in the shape of an arc, which is formed on a coordinated surface of the rotation control means with the end member, having a first inscribed angle about the axis of rotation, and further being movably linked with a first protrusion formed in the end member; and second linking grooves in the shape of an arc, which are formed on a coordinated surface of the rotation control means with the rotary body, having a second inscribed angle around the axis of rotation, and further being movably linked with second protrusions formed in the rotary body; wherein a maximum rotated angle of the rotary body about the end member is larger than or equal to 360 degrees.

The present invention provides a rotary support structure for medical equipment by which a piece of medical equipment such as a surgical microscope can be rotated for more than 1 turn and also can be stopped at an angle of rotation having no bad effect on the inner wirings.

According to a second technical aspect of the present invention, it is characterized in that additionally, in the rotary support structure for medical equipment, the rotation control means has a first rotary plate and a second rotary plate which are rotatable at least about the axis of rotation; the first linking groove is formed on the coordinated surface of the first rotary plate with the end member; the second linking groove is formed on the coordinated surface of the second rotary plate with the rotary body; a third linking groove, in the shape of an arc, having a third inscribed angle around the axis of rotation is formed on a coordinated surface of one of the rotary plates with the other adjacent rotary plate; and a third protrusion being linking movable with the third linking groove is formed on a coordinated surface of the adjacent rotary plate with the third linking groove.

### BRIEF DESCRIPTION OF THE DRAWINGS

Fig.1 is a side view showing a stand system according to a first embodiment of the present invention;
Fig.2 is a side view showing a state where a rotary body is off an end member of the stand system;
Fig.3 is a plan view showing a rotary plate attached to a bottom of the end member;
Fig.4 is a cross-sectional view, which is viewed in the direction of SA-SA arrowed in Fig.3;
Fig.5 is an exploded view of Fig.4;
Fig.6 is a longitudinal cross-sectional view of the rotary plate;
Fig.7 is a lateral cross-sectional view of the rotary plate showing a first groove and a second groove;
Fig.8 is a lateral cross-sectional view of the rotary plate showing a modified example of a first groove and a second groove thereof; and
Fig.9 is a longitudinal cross-sectional view showing a rotary plate according to a second embodiment.

### DESCRIPTION OF THE PREFERRED EMBODIMENT

In the following, the preferred embodiments of the present invention will be described referring to the drawings.

### First Embodiment

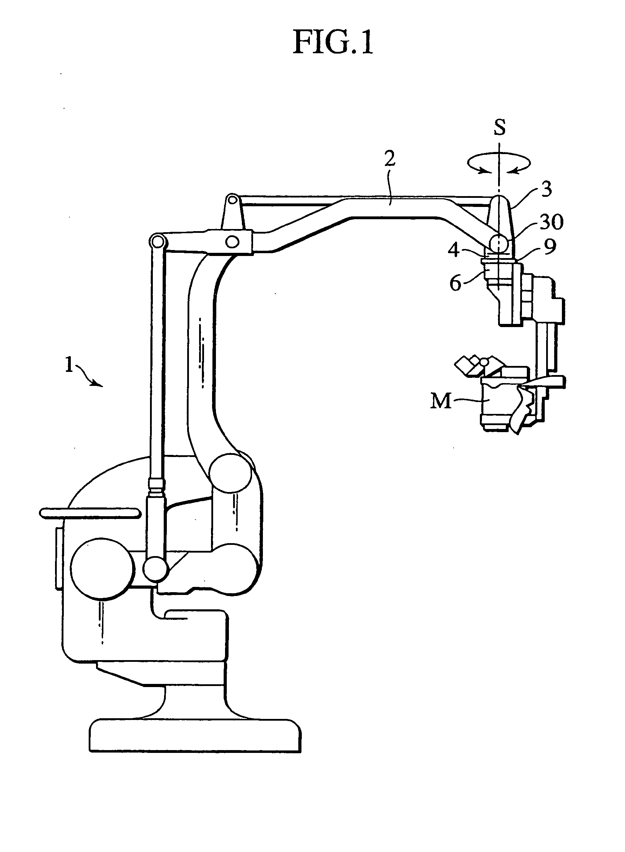

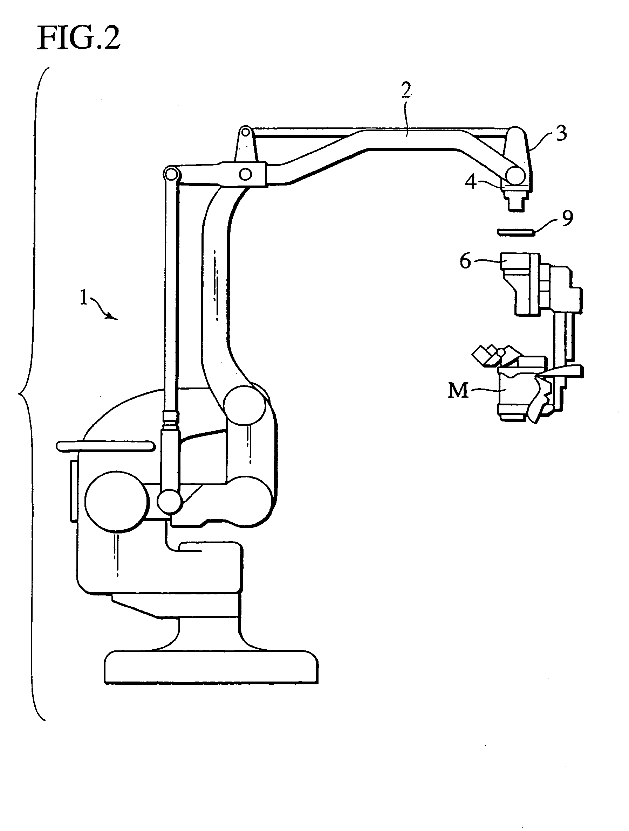

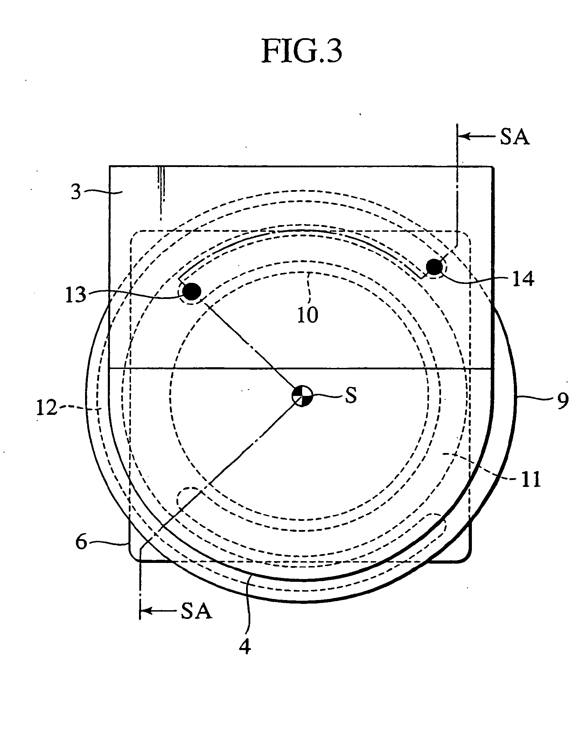

Fig.1- Fig.7 are views showing a first embodiment of the present invention. A support arm 2 is disposed at the upper portion of a stand system 1 and extends laterally. An end member 3 is disposed at the end of the support arm 2 and is always maintained vertically by a linkage mechanism.

A lower end portion 4 of the end member 3 in the present embodiment is slightly bent in the lateral direction. A tubular portion 5, the outer diameter of which varies stepwise in the direction of an axis of rotation S, is formed downward from the lower end portion 4. A rotary body 6 in the shape of a quadrilateral box is attached rotatable about the axis of rotation S to the tubular portion 5. A surgical microscope M is attached to the rotary body 6. Additionally, the axis of rotation S is a theoretical axis and the rotary body 6 rotates about the axis.

The tubular portion 5 makes the rotary body 6 freely rotatable by letting a couple of an upper and lower bearings 7, 8 lie between the tubular portion and the rotary body 6 in a state where the tubular portion passes through the midair portion of the rotary body 6. The rotary body 6 attached only to the tubular portion 5 is rotatable without restriction around the axis of rotation S by a function of the bearings 7, 8.

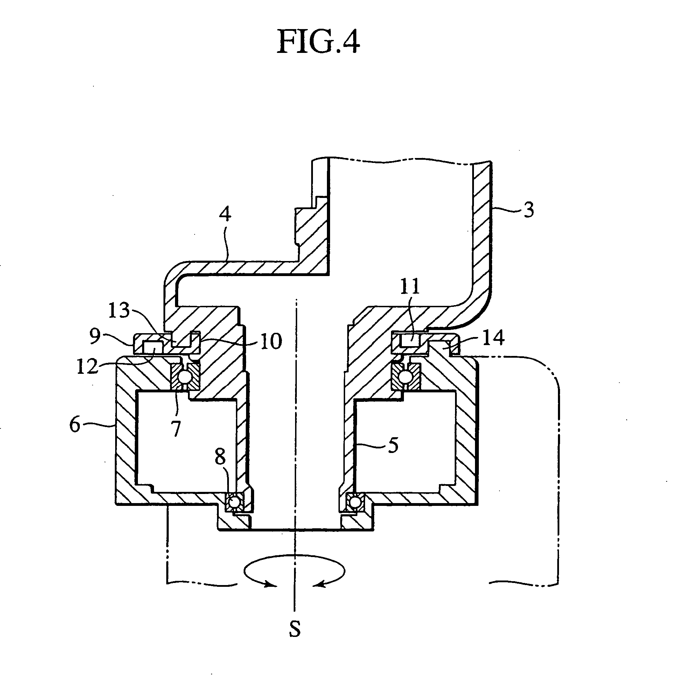

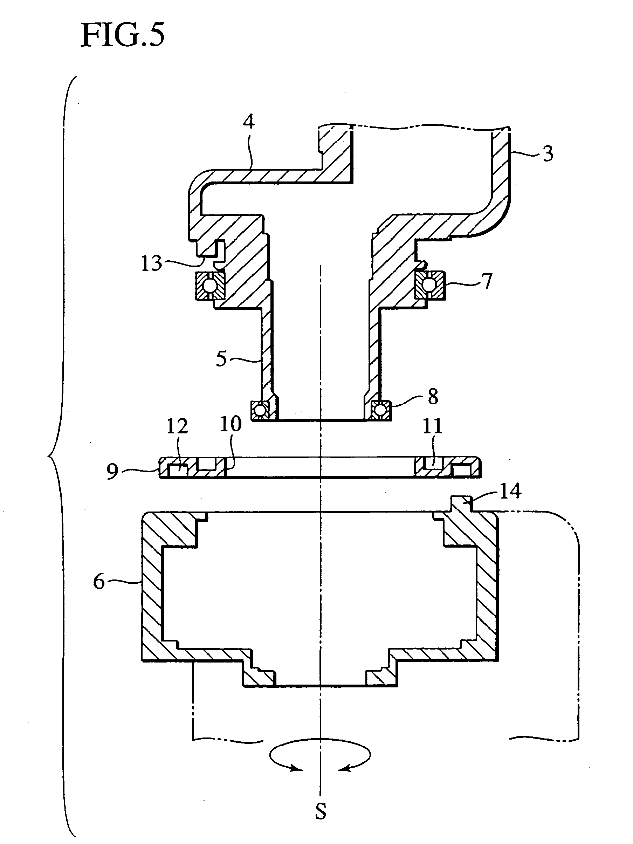

Besides, although the tubular portion 5 and the rotary body 6 are shown as one-pieces respectively in Fig.4 and Fig.5, they practically comprises a plurality of components, and mounting of the bearings 7, 8 or installation of the rotary body 6 can be achieved by means of assembling a plurality of components.

Between the lower end portion 4 of the end member 3 and the rotary body 6 provided is one rotary plate 9 as a countermeasure against binding or tangling for restricting an angle of rotation of the rotary body 6. The rotary plate 9 is in the shape of a circular plate having a substantially circular-through-hole 10 through which the tubular portion 5 is inserted in the central portion thereof. The rotary plate 9 is supported rotatable in a state where a hypothetical center of the circle existing in the through-hole 10 is coincided with the axis of rotation S.

A first groove 11 in the shape of an arc, which has an inscribed angle (a first inscribed angle θ1) of 270° (3/4 turn) about the axis of rotation S, is formed on a coordinated surface (a top surface) of the rotary plate 9 with the lower end portion 4 of the end member 3. A second groove 12 in the shape of an arc, which has an inscribed angle (a second inscribed angle θ2) of 270° (3/4 turn) around axis of rotation S, is formed on a coordinated surface (a bottom surface) of the rotary plate 9 with the rotary body 6. Each of the minor arc regions of the first groove 11 and the second groove 12 (major arcs) is not grooved to serve as a stopper and are preferably disposed substantially in axial symmetry with each other.

Since the first groove 11 and the second groove 12 are configured to be different from each other in terms of radii of the arcs and the second groove 12 is larger in diameter than the first groove 11, the first groove 11 and the second groove 12 are never superimposed on each other in the thickness direction of the rotary plate 9. Therefore, the thickness of the rotary plate 9 can be thinner than the sum total of a depth of the first groove 11 and that of the second groove 12. A first protrusion 13 in the shape of an arc is formed on a bottom surface (a surface coordinated with the rotary plate 9) of the lower end portion 4 of the end member 3, and the first protrusion 13 is linked to be movable within the first groove 11. Further, a second protrusion 13 in the shape of an arc is formed on a top surface (a coordinated surface with the rotary plate 9) of the rotary body 6, and the second protrusion 14 is linked to be movable within the second groove 12.

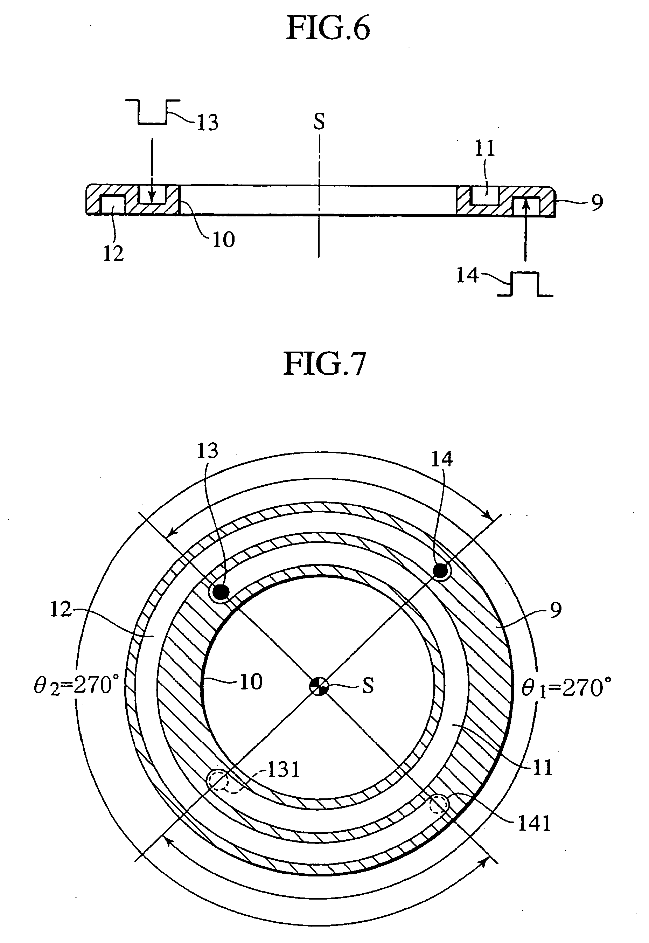

According to the embodiment, both the end member 3 and the rotary body 6 become rotatable relative to the rotary plate 9 within an inscribed angle of 270° (3π/2 radians) or less. That is to say, in Fig.7, when the second protrusion 14 is rotated counterclockwise making the first protrusion 13 and the rotary plate 9 stay stationary temporarily, it will move to a position designated as 141 by a turn of an angle θ1. Further, when the second protrusion is rotated together with the rotary plate 9 counterclockwise by an angle θ2, the first protrusion will move to a position, which is designated as 131 and the rotation of the second protrusion will be restricted. Consequently, a maximum rotated angle Ω of the rotary body 6 is set as an angle of θ1+θ2. The situation is the same when the second protrusion 14 is rotated clockwise.

With regard to the rotary body 6 for supporting a surgical microscope M, it is rotatable up to Ω=540° (3π radians) at a maximum angle and the rotary body 6 can be stopped after a turn of 540°. Thereby, both easy handling of the surgical microscope by a surgeon and protection of cables within the rotary support portion can be combined using a simple structure.

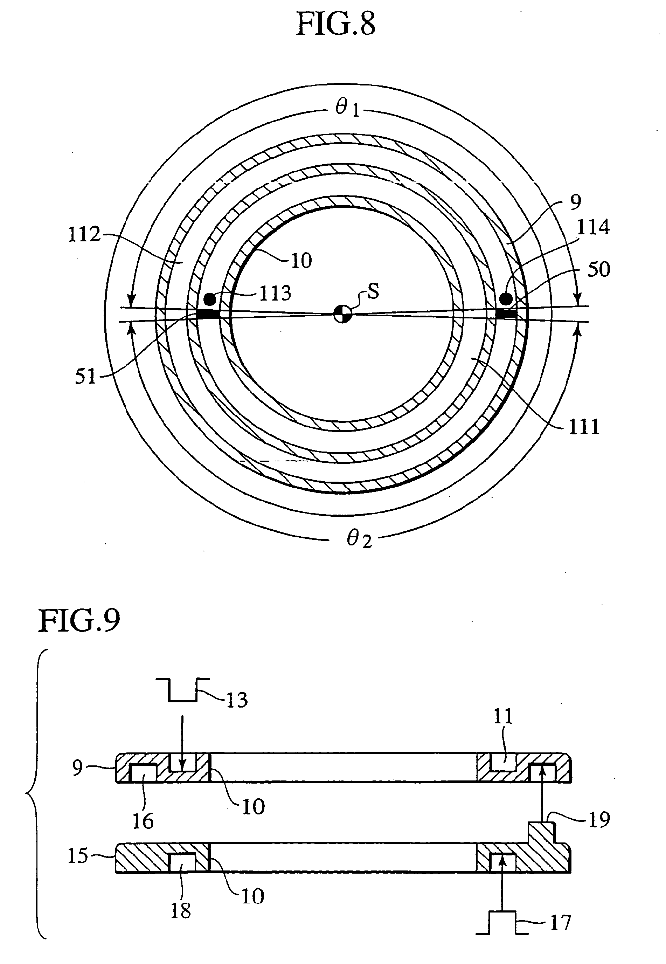

When a first groove 111 and a second groove 112 are configured to be in the shape of a ring respectively and stopper members 50, 51 are disposed in each ring respectively as shown in Fig.8, an angle of rotation of each groove will be enlarged up to approximately 360°.

It has been described in the present embodiment that the rotary plate 9 is usually attached to the coordinated portion of the end member 3 of the support arm 2 with the rotary body 6, however the present invention is not limited thereto but can be applied to the rotary plate which is inserted between the 2 members coordinated with each other so as to be rotatable.

### Second Embodiment

Fig.9 shows a second embodiment of the present invention. The following description will be made putting like reference numerals and signs to portions corresponding with those in the first embodiment.

In the second embodiment, two rotary plates 9, 15 are disposed on a coordinated portion between a lower end portion 4 of an end member 3 and a rotary body 6. A shape of the upper rotary plate 9 is the same as that of the first embodiment, however a groove on a bottom surface is used as an auxiliary groove 16. A first protrusion 13 of the end member 3 is linked to be movable within a first groove 11 similarly to the first embodiment.

On a bottom surface of the lower rotary plate 15, a second groove 18 is formed configured to engage with a second protrusion 17 formed on the rotary body 6 and linked to be movable therewith. Diameters of the first groove 11 and that of the second groove 17 are the same and the grooves are superimposed on each other in the up-and-down direction (the direction of each thickness of the rotary plates). Therefore, the second protrusion 17 is positioned further inward than that in the first embodiment. On a top surface of the lower rotary plate 15, an auxiliary protrusion 19 is formed configured to engage with an auxiliary groove 16 of the upper rotary plate 9 and linked to be movable therewith. All of the first groove 11, the auxiliary groove 16 and the second groove 18 are in the shape of an arc having an inscribed angle of 270° (3/4 turns) around an axis of rotation S.

In the second embodiment, the maximum angle Ω of the rotary body 6 will be 810° (9/4 turns) by employing the two rotary plates 9, 15. Consequently, a surgical microscope M can be rotated up to 810° and the rotary-body 6 can be stopped after the turn of 810°. Ease of handling the surgical microscope M is further enhanced due to a larger angle of rotation than that in the first embodiment.

Additionally, the auxiliary groove 16 and the auxiliary protrusion 19 can be substituted for each other. The number of the rotary plates can be increased as long as there occurs no problem of inner wirings. If the number of the rotary plates is set to be N, a maximum rotated angle of the rotary body 6 can be 270° × (N + 1) when an angle of rotation of each groove is also set to be 270°.

Further, when an inscribed angle of each groove is set to be 360°, a maximum rotated angle of rotation of the rotary body 6 can be enlarged up to approximately 360°×(N + 1).

A maximum rotated angle the rotary body 6 can be adjusted to any desired amount by adjusting each inscribed angle (a length of an arc) about the axis of rotation S of the first roove 11, the second grooves 12, 18 and the auxiliary groove 16 in the first embodiment and in the second embodiment.

As described above, according to the present invention, a surgical microscope can be rotated for more than 1 turn and also can be stopped after a number of turns having no bad effect on the inner wirings. Therefore, both easy handling of the surgical microscope and protection of inner wirings can be combined employing a simple structure.

Although the invention has been described above by reference to certain embodiments of the invention, the invention is not limited to the embodiments described above. Modifications and variations of the embodiments described above will occur to those skilled in the art, in light of the teachings. The scope of the invention is defined with reference to the following claims.

## Claims

1. A rotary support structure for medical equipment, in which a rotary body (6) being rotatable about a theoretical axis of rotation (S) is attached onto an end member (3) of a support arm (2) of a stand system (1) and a medical equipment (M) is supported thereon by the rotary body, comprising:
rotation control means (9, 15) being disposed between the end member and the rotary body and being rotatable about the rotational axis,
wherein a maximum rotated angle of the rotary body about the end member is larger than or equal to 360 degrees, **characterized in that** the structure further comprises a first linking groove (11) in the shape of an arc formed on a coordinated surface of the rotation control means with the end member, the first linking groove having a first inscribed angle about the rotational axis and being movably linked with a first protrusion (13) formed in the end member, and
a second linking groove (12, 18) in the shape of an arc formed on a coordinated surface of the rotation control means with the rotary body, the second linking groove having a second inscribed angle about the rotational axis and being movably linked with a second protrusion (14, 17) formed in the rotary body.

2. The rotary support structure for a medical equipment of claim 1, wherein the rotation control means is a rotary plate (9).

3. The rotary support structure for medical equipment of claim 1, wherein
the rotation control means (9, 15) has a first rotary plate and a second rotary plate which are rotatable at least about the rotational axis,
the first linking groove (11) is formed on the coordinated surface of the first rotary plate (9) with the end member,
the second linking groove (18) is formed on the coordinated surface of the second rotary plate (15) with the rotary body,
a third linking groove (16) in the shape of an arc is formed on a coordinated surface of each of the rotary plates with the other adjacent rotary plate and has a third inscribed angle about the rotational axis, and
a third protrusion (19) is formed on a coordinated surface of the adjacent rotary plate with the third linking groove and is movably linked with the third linking groove.

4. The rotary support structure for medical equipment of claim 2, wherein a diameter of each arc of the first linking groove and the second linking groove are different from each other in order for the grooves not to be superimposed on each other in the thickness direction of the rotary plate.

5. The rotary support structure for medical equipment of claim 3, wherein
a diameter of each arc of the first linking groove (11) and the third linking groove (16), which are formed on both surfaces of the first rotary plate (9), are different from each other in order for the grooves not to be superimposed on each other in the thickness direction of the first rotary plate, and
a diameter of each arc of the second linking groove (18) and the third protrusion (19), which are formed on both surfaces of the second rotary plate (15), are different from each other in order for the grooves not to be superimposed on each other in the thickness direction of the first rotary plate.

## Patentansprüche

1. Drehbare Tragstruktur für medizinische Einrichtungen, bei der ein um eine theoretische Drehachse (S) drehbarer Drehkörper (6) an einem Endteil (3) eines Tragarms (2) eines Ständersystems (1) befestigt und eine medizinische Einrichtung (M) daran durch den Drehkörper getragen ist, umfassend:
Drehungssteuemilttel (9, 15), die zwischen dem Endteil und dem Drehkörper angeordnet und um die Drehachse drehbar sind,
wobei ein maximaler Drehwinkel des Drehkörpers um das Endteil größer als oder gleich 360 Grad ist, **dadurch gekennzeichnet, dass** die Struktur weiter umfasst:
eine erste Verbindungsrinne (11) in Form eines Bogens, der an einer koordinierten Oberfläche der Drehungssteuermittel mit dem Endteil ausgebildet ist, wobei die erste Verbindungsrinne einen ersten einbeschriebenen Winkel um die Drehachse aufweist und bewegbar mit einem ersten, in dem Endteil ausgebildeten Vorsprung (13) verbunden ist, und
eine zweite Verbindungsrinne (12, 18) in Form eines an einer koordinierten Oberfläche der Drehungssteuermittel mit dem Drehkörper gebildeten Winkels, wobei die zweite Verbindungsrinne einen zweiten einbeschriebenen Winkel um die Drehachse aufweist und bewegbar mit einem zweiten, in dem Drehkörper ausgebildeten Vorsprung (14, 17) verbunden ist.

2. Drehbare Tragstruktur für eine medizinische Einrichtung nach Anspruch 1, bei der das Drehungssteuermittel eine Drehplatte (9) ist.

3. Drehbare Tragstruktur für medizinische Einrichtung nach Anspruch 1, bei der
das Drehungssteuermittel (9, 15) eine erste Drehplatte und eine zweite Drehplatte aufweist, die mindestens um die Drehachse drehbar sind,
die erste Verbindungsrinne (11) an der koordinierten Fläche der ersten Drehplatte (9) mit dem Endteil ausgebildet ist,
die zweite Verbindungsrinne (18) an der koordinierten Fläche der zweiten Drehplatte (15) mit dem Drehkörper ausgebildet ist,
eine dritte Verbindungsrinne (16) in Form eines Bogens an einer koordinierten Fläche jeder der Drehplatten mit der anderen benachbarten Drehplatte ausgebildet ist und einen dritten einbeschriebenen Winkel um die Drehachse aufweist, und
ein dritter Vorsprung (19) an einer koordinierten Fläche der benachbarten Drehplatte mit der dritten Verbindungsrinne ausgebildet und bewegbar mit der dritten Verbindungsrinne verbunden ist.

4. Drehbare Tragstruktur für medizinische Einrichtung nach Anspruch 2, bei der der Durchmesser jedes Bogens der ersten Verbindungsrinne und der zweiten Verbindungsrinne verschieden voneinander sind, damit die Rinnen in Stärkerichtung der Drehplatte nicht übereinander angeordnet werden.

5. Drehbare Tragstruktur für medizinische Einrichtung nach Anspruch 3, bei der
der Durchmesser jedes Bogens der ersten Verbindungsrinne (11) und der dritten Verbindungsrinne (16), die an beiden Oberflächen der ersten Drehplatte (9) ausgebildet sind, voneinander verschieden sind, damit die Rinnen in Stärkerichtung der ersten Drehplatte nicht übereinander angeordnet werden, und
der Durchmesser jedes Bogens der zweiten Verbindungsrinne (18) und der dritte Vorsprung (19), die an beiden Oberflächen der zweiten Drehplatte (15) ausgebildet sind, voneinander verschieden sind, damit die Rinnen in Richtung der Stärke der ersten Drehplatte nicht übereinander angeordnet werden.

## Revendications

1. Structure rotative de support pour appareillage médical, dans laquelle un corps (6) rotatif pouvant tourner autour d'un axe (S) théorique de rotation est attaché sur un élément (3) d'extrémité d'un bras (2) de support d'un système (1) de présentoir et un appareil médical (M) y est supporté par le corps rotatif, comprenant:
des moyens (9,15) de commande de la rotation étant situés entre l'élément d'extrémité et le corps rotatif et pouvant tourner autour de l'axe de rotation,
dans lequel un angle de rotation maximum du corps rotatif autour de l'élément d'extrémité est supérieur ou égal à 360 degrés, **caractérisé en ce que** la structure comprend en outre une première rainure (11) de liaison en forme d'arc formée sur une surface associée aux moyens de commande de la rotation avec l'élément d'extrémité, la première rainure de liaison ayant un premier angle inscrit autour de l'axe de rotation et étant reliée en pouvant se déplacer à une première protrusion (13) formée dans l'élément d'extrémité, et
une deuxième rainure (12, 18) de liaison en forme d'arc formée sur une surface associée au moyen de commande de la rotation avec le corps rotatif, la deuxième rainure de liaison ayant un deuxième angle inscrit autour de l'axe de rotation et étant reliée en pouvant se déplacer à une deuxième protrusion (14, 17) formée dans le corps rotatif.

2. Structure rotative de support pour appareillage médical selon la revendication 1, dans laquelle le moyen de commande de la rotation est une plaque (9) rotative.

3. Structure rotative de support pour appareillage médical selon la revendication 1, dans laquelle les moyens (9, 15) de commande de la rotation sont une première plaque rotative et une seconde plaque rotative qui peuvent tourner au moins autour de l'axe de rotation,
la première rainure (11) de liaison est formée sur la surface associée à la première plaque (9) rotative et à l'élément d'extrémité,
la deuxième rainure (18) de liaison est formée sur la surface associée à la deuxième plaque (15) rotative et au corps rotatif,
une troisième rainure (16) de liaison en forme d'arc est formée sur une surface associée à chacune des plaques rotatives avec l'autre plaque rotative adjacente et comporte un troisième angle inscrit autour de l'axe de rotation, et
une troisième protrusion (19) est formée sur une surface associée à la plaque rotative adjacente et à la troisième rainure de liaison et est reliée en pouvant se déplacer avec la troisième rainure de liaison.

4. Structure rotative de support pour appareillage médical selon la revendication 2, dans laquelle les diamètres de chaque arc de la première rainure de liaison et de la deuxième rainure de liaison sont différents l'un de l'autre afin que les rainures ne se superposent pas l'une l'autre dans la direction de l'épaisseur de la plaque rotative.

5. Structure rotative de support pour appareillage médical selon la revendication 3, dans laquelle
les diamètres de chacun des arcs de la première rainure (11) de liaison et de la troisième rainure (16) de liaison, qui sont formées sur les deux surfaces de la première plaque (9) rotative, sont différents l'un de l'autre afin que les rainures ne se superposent pas l'une l'autre dans la direction de l'épaisseur de la première plaque rotative, et
les diamètres de chacun des arcs de la deuxième rainure (18) de liaison et de la troisième protrusion (19), qui sont formées sur les deux surfaces de la deuxième plaque (15) rotative, sont différents l'un de l'autre afin que les rainures ne se superposent pas l'une l'autre dans la direction de l'épaisseur de la première plaque rotative.
